# EUROPEAN PATENT SPECIFICATION

(11) **EP 3 817 568 B1**
(45) Date of publication and mention of the grant of the patent: **13.09.2023**
(21) Application number: 19831304.1
(22) Date of filing: 06.07.2019
(51) Int. Cl.: A23D 9/02, C11C 3/10, C07C 67/48, C07C 69/587

(54) **METHOD FOR OBTAINING PURIFIED FATTY ACID ESTER COMPOSITION AND FATTY ACID ESTER COMPOSITION**
VERFAHREN ZUR HERSTELLUNG EINER GEREINIGTEN FETTSÄUREESTERZUSAMMENSETZUNG UND FETTSÄUREESTERZUSAMMENSETZUNG
PROCEDE D'OBTENTION D'UNE COMPOSITION D'ESTER D'ACIDE GRAS PURIFIEE ET COMPOSITION D'ESTER D'ACIDE GRAS

(30) Priority: 06.07.2018 PL 42622718
(43) Date of publication of application: 12.05.2021
(73) Proprietor: onesano S.A., 41-506 Chorzow (PL)
(72) Inventor: DU Y, Sebastian, 43-140 L dziny (PL); KWA NIAK, Wojciech, 43-518 Ligota (PL)
(74) Representative: Witek, Rafal
(86) International application number: PCT/PL2019/050037
(87) International publication number: WO 2020/009593

(56) References cited:
- WO-A1-2005/070411
- WO-A1-2005/070411
- JP-A- 2011 046 834
- JP-A- 2011 046 834
- PL-B1- 228 103
- PL-B1- 228 103

## Description

The first object of the invention is a method for obtaining a purified composition of higher fatty acid esters (hereinafter HFA) substantially free of undesirable and harmful impurities of heavy metals and dioxins, which occur in crude unsaturated oils of natural origin used for the production of esters. The second object is the purified composition of ethyl esters of unsaturated fatty acids of natural origin, i.e. of fish and vegetable origin, selected from the group consisting of: ALA (α-linolenic acid), DHA (docosahexaenoic acid) and EPA (eicosapentaenoic acid), which composition can be obtained by the method of the invention. The invention finds application in the technological process of producing higher fatty acid esters using the transesterification reaction, where it is necessary to purify the reaction products from alcohol and impurities that adversely affect their quality in use.

Fatty oils are substances of natural origin. Their synthesis takes place in living organisms (plants and animals) that exist in the natural environment. As a result, natural oils are mixtures of metabolites of living organisms and their accompanying substances. They can be dyes, proteins and sugars, which are also formed as a result of biosynthesis. Such natural mixtures also contain other substances that penetrate them from the environment, including harmful environmental pollutants. Therefore, fatty acid glycerides are naturally accompanied by other compounds that from a chemical and functional point of view are undesirable impurities. Therefore, purifying and obtaining chemically pure compounds from natural mixtures is not a trivial issue. In most cases, it is not necessary to obtain chemically pure mixtures for food purposes. In this case, it is important to get rid of substances harmful to health. In this context, heavy metals and their compounds are particularly undesirable. These are substances that naturally penetrate living organisms from a contaminated environment. They show harmful effects to health, even in small quantities. It is therefore important to remove them, especially in the case of the production of ingredients used in the manufacturing of pharmaceutical preparations or dietary supplements, whose task is to support health.

According to the official position of the European Food Safety Authority /European Food Safety Authority, 2008 Draft consolidated list of Health Claims/ diet containing the correct proportion of ω-6 to ω-3 acids contributes to a number of pro-health effects. The correct proportions: support the work of the heart muscle, normalize blood pressure, are anticoagulant, are antiatherosclerotic, by reducing triglyceride levels they help to maintain normal cholesterol level and improve fat metabolism, exert beneficial effects on cognitive functions, protect the cardiovascular system at the cellular level, are necessary for the proper functioning of the retina of the eye, have anti-inflammatory, antiallergic, antidepressant properties, counteract obesity, ensure optimal brain development and functioning.

A composition is known from patent application P.408510 that stimulates the proliferation of mesenchymal stem cells and contains a mixture of ethyl esters of unsaturated fatty acids of plant origin obtained as a result of transesterification of selected vegetable oils with ethanol. The resulting mixture of esters is purified by means of a sedimentation tanks system and a purification centrifuge. However, the use of several stages of mechanical or gravitational cleaning does not allow complete removal of trace amounts of heavy metals, which raw materials are contaminated with, and soaps formed during the production process (transesterification reaction), adversely affecting taste.

A method for producing a mixture of ethyl esters of plant fatty acids with a high content of cis unsaturated fatty acids is known from patent application P.408509. The production method described in this document includes the step of removing catalyst, soaps and glycerin. However, heavy metals found in initial raw materials are not taken into account.

Document US8829048B2 discloses a composition for the treatment of central nervous system disorders containing fatty acids such as: ALA (a-linolenic acid) or DHA (docosahexaenoic acid) or EPA (eicosapentaenoic acid) in a mixture with DHA [column 3, lines 56-67]. The acid content in the composition is preferably 85%. The preferred forms are alkyl esters, methyl to ethyl (preferably ethyl) esters, glycerides and salts, e.g. potassium or sodium. The substrates are obtained from natural sources such as fish oil, algae or vegetable oil. The document does not describe the process of esterification itself and purification (of products or substrates).

Solutions for purifying fish oils from heavy metal compounds are known in the art. However, they require a number of operations that significantly increase the cost of producing dietary supplements.

Patent application US20110244052A1 discloses a composition containing polyunsaturated fatty acids or derivatives thereof for use in the treatment of vision defects. The primary source of acids is fat obtained from squid liver. The composition contains EPA and DHA. The purification process is disclosed in the second example. The frozen liver is freeze-dried, then it is extracted with supercritical carbon dioxide. The liver is further washed with ethanol to wash out the remaining lipids. The combined fractions are subjected to vacuum distillation and purification on a sorbent. The disclosed process involves a technologically complex supercritical extraction and vacuum distillation operation. Despite the considerable complexity of the technological process, the operations introduced do not guarantee the removal of adverse trace components (especially heavy metals).

Document US9163198B2 discloses a method for purifying EPA (eicosapentanoic) ethyl ester from crude fish oil using a combination of new esterification techniques and a simulated moving bed (SMB). Crude fish oil diluted in a nonpolar solvent (heptane) is esterified with an acid and then transesterified with a base. After washing, the non-polar phase is directly passed to the SMB zone, including normal phase separation using a hydrophilic stationary phase agent and a non-polar/organic polar mobile phase desorbent to provide an improved omega-3 product containing EPA. In another embodiment, the improved omega-3 product is passed to the second and third SMB zones operating in reverse phase using a hydrophobic stationary phase and a polar mobile phase desorbent. The method enables EPA to be produced with a high purity of 97% wt. However, it only applies to one of the acids - EPA. In addition, the non-polar solvent (heptane) used may also be included (in trace form) in the product. The document does not mention purification of the product, at any stage, from traces of heavy metals.

US9694302B2 discloses a chromatographic separation process for recovering polyunsaturated fatty acid from a batch mixture, which includes: (a) purifying the batch mixture in a first chromatographic separation step using a mixture of water eluent and a first organic solvent to obtain an intermediate product; and (b) purifying the intermediate product in a second chromatographic separation step using a mixture of water and a second organic solvent as eluent to obtain the product. Organic solvents differ in polarity. The invention relates to the separation of EPA from ALA and GLA and its purification. The source of EPA is fish oil. The inventors do not refer at all to the issue of heavy metal content in raw materials and final products.

In the light of the state of the art, there is still a need to solve the problem of reducing heavy metal content to a level acceptable by standards, which would allow the use of unsaturated fatty acids obtained from natural resources, especially from oils obtained from plants or fish, in dietetic or medicinal products. The desired method should allow purification during the technological process without the need for agents that may additionally contaminate the derivatives of unsaturated fatty acids (especially esters) and adversely affect their taste. In the prior art, EPA is mainly purified, while other natural unsaturated fatty acids or their derivatives, such as DHA or ALA, are not. Known solutions also do not allow the purification of mixtures of various acids and their derivatives. PL 228 103 discloses a method of producing a mixture of ethyl esters of plant fatty acids with a high content of cis- isomers of unsaturated fatty acids where the mixture of plant oils is used as a feedstock in the transesterification reaction with ethanol and after completing the transesterification reaction, ethanol is evaporated out of the reaction mixture.

JP2011046834 teaches a method for producing fatty acid alkyl esters.

Unexpectedly, these technical problems were solved by the present invention.

The first object of the invention is a method for the preparation of a purified composition containing unsaturated fatty acid esters obtained from a transesterification reaction of a natural oil containing unsaturated fatty acid triglycerides with ethyl alcohol, characterized in that the purification of the reaction mixture is carried out in the following stages:
a) after the transesterification reaction, the alcohol is evaporated from the reaction mixture, wherein the evaporation process is carried out at a temperature not exceeding 49 °C for not more than an hour,
b) the ester phase obtained in step (a) is separated,
c) the ester phase is purified from catalyst residues, soaps and glycerin in the centrifugation process,
d) the ester phase is mixed with water in a volume ratio of 1:1, with nitrogen gas flow through the resulting two-phase system at 5-10 m³/h for one hour, then the nitrogen flow is stopped and the mixture is left to separate into two phases, then the aqueous phase is separated.
e) the purified ester mixture is clarified by centrifugation.

Preferably, the evaporation is carried out in a two-stage evaporative module.

Preferably, the ester phase with water is mixed in the 24-hours separator.

Preferably, fish or vegetable oils or mixtures thereof are used to produce esters.

The second subject of the invention is a purified composition containing unsaturated fatty acids ethyl esters obtained by the transesterification reaction of a natural oil containing unsaturated fatty acid triglycerides with ethyl alcohol, characterized in that it contains, and preferably is, a mixture of fatty acid ethyl esters with the following omega fatty acids composition:
- alpha-linolenic acid (ALA) ethyl ester in an amount of 1% to 50% wt. ,
- eicosapentaenoic acid (EPA) ethyl ester in an amount of 1% to 40% wt. ,
- docosahexaenoic acid (DHA) ethyl ester in an amount of 1% to 40% wt. ,
wherein it is essentially free of dioxins, biphenyls, their derivatives and heavy metals contaminants and contains a sum of dioxins (denoted as WHO-PCDD/F-TEQ) in an amount not exceeding 0.158 pg/g fat, the sum of polychlorinated biphenyls (denoted as WHO-PCB-TEQ) in an amount not exceeding 0.159 pg/g fat, the sum of polychlorinated dioxins and biphenyls (denoted as WHO-PCDD/F-PCB-TEQ) in an amount not exceeding 0.317 pg/g fat, the sum of polychlorinated biphenyls (denoted as the sum of PCBs (ICES - 6)) in an amount not exceeding 14.67 ng/g fat, and heavy metals selected from the group consisting of: arsenic, cadmium, lead, mercury, with arsenic in an amount not exceeding 0.01 mg/kg, cadmium in an amount not exceeding 0.001 mg/kg, lead in an amount not exceeding 0.015 mg/kg, mercury in an amount not exceeding 0.0006 mg/kg.Preferably, the composition according to the invention contains ethyl esters of unsaturated fatty acids derived from a mixture of fish and vegetable oil.

During the production of ethyl esters from fish oils by the alkaline transesterification method according to the invention, it was surprisingly found that the content of heavy metal compounds in the finished product is significantly lower than in the source oil, which is the raw material for production. The effect was surprising because some heavy metals, including mercury, form organometallic compounds which are well soluble in fat. The chemical nature of ethyl esters and glycerides of higher fatty acids is similar, therefore fat-soluble compounds would be expected to dissolve similarly in glycerides and ethyl esters. Indeed, studies on the solubility of methylmercury in oils of vegetable or animal origin, including in particular those of fish oils and ethyl esters of these oils, have shown that the solubility of this compound in the above media is similar.

Therefore, the reasons for the different concentration of heavy metal compounds in ethyl esters of higher fatty acids and the source oil from which they were made should be sought in the very process of production of ethyl esters. It turned out that there are significant differences in the concentration of heavy metals found in the glycerin phase obtained in the production process, in relation to both esters and base oil, which is the raw material for the synthesis. This suggests that the glycerol phase formed in the production process acts as an extractant for heavy metal compounds, both organometallic and simple salts of these metals. It can be assumed that this is due to the specific composition of the glycerin phase - containing both hydrophobic and hydrophilic compounds. It is likely that the water contained in the glycerin phase also contributes to the extraction of heavy metal salts. In the case of organometallic compounds, the observed effect should also be associated with the presence of free fatty acids and their soaps. It is also possible that more complex substances contained in trace amounts, such as dyes and proteins, chelate heavy metals, causing their removal from the ester phase (the final reaction product).

The invention also aimed to improve the organoleptic qualities of the esters obtained.

The search for a way to improve the taste of ethyl esters of higher fatty acids of plant origin, including blends of linseed oil, blackcurrant and evening primrose known from patent application P.408 510, directed the inventors towards the extraction with water used in the production of HFA methyl esters as biofuels. In the case of this technology, the HFA soaps washing out process is used to remove them from the reaction system at the end of the methanol evaporation process. These soaps catalyze the reaction reversed to transesterification. Therefore, for complete removal of methanol from the reaction mixture, it is first necessary to remove the system that catalyses this reaction. In the research preceding the invention, a similar procedure was applied to the product (purified by mechanical methods - decantation, centrifugation) of alkaline transesterification of a mixture of ethyl esters of EPA, DHA and ALA acids. Surprisingly, also in the ethyl alcohol based reaction system, water extraction allowed the soap to be removed from the reaction system. As a result, a significant improvement in taste was achieved, in particular the characteristic bitterness of HFA ethyl esters was removed, probably due to the trace content of potassium soaps, which are not removed from the reaction system by mechanical methods. Mechanical separation only removes the phase immiscible with HFA ethyl esters from the system, i.e. the glycerol phase. The limit concentration of glycerol phase components is determined by their solubility in a mixture of ethyl esters. It is not possible to remove them by centrifugation or gravitational separation. Their concentration in the product is very low, but their taste is palpable. The use of water extraction reduced the content of glycerol phase components in the synthesis product.

At the same time, the content of heavy metals in the final product was reduced. Extraction of the ester phase (final product) with water turned out to have positive effects both in terms of taste improvement, which is important from the practical point of view, as well as in reducing the amount of heavy metals in the product. Finally, after centrifugation of the ester phase from water, a clear solution of a mixture of fish oil HFA ethyl esters was obtained, with a reduced characteristic odor of fish, reduced bitterness and a lower heavy metals content than obtained by other available methods.

In this way, a product with pro-health properties unique on the market is obtained from natural EPA, DHA and ALA triglycerides containing harmful to health impurities of heavy metal compounds, especially mercury, cadmium, arsenic and lead, after processing them into a simple form of ethyl esters by alkaline trans-esterification and water extraction. The proposed method reduces the total concentration of heavy metals on average by 25 times. Particularly good effects were obtained when the concentration of lead was lowered.

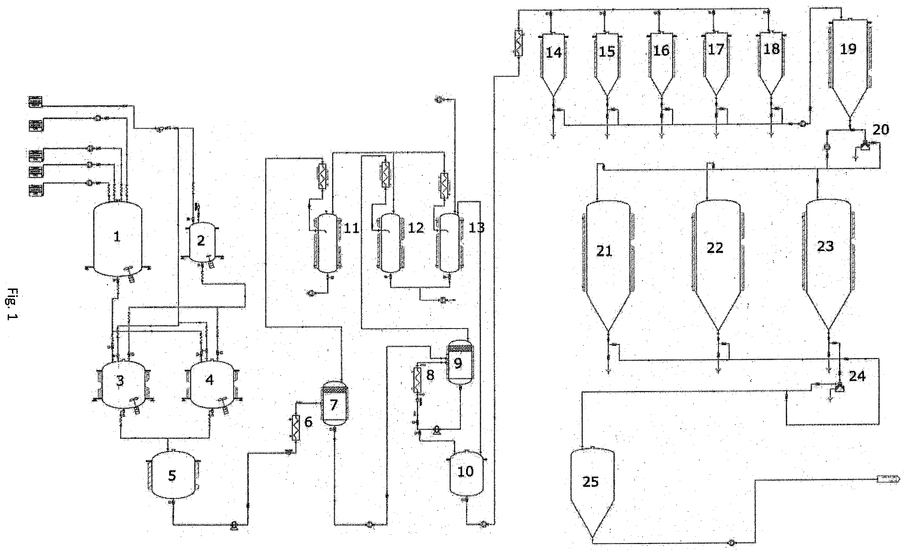

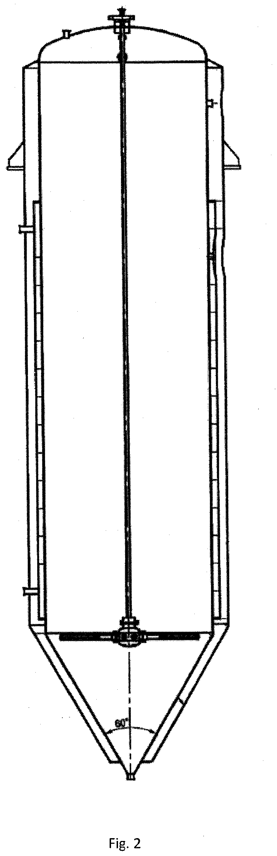

Examples of the embodiment of the inventions are illustrated in Fig. 1, which shows a technological diagram of the ester composition purification, where: 1. Oil pre-mixing tank, 2. Catalyst mixer (catalyst preparation tank), 3. 4. Reactor, 5. Buffer tank for first evaporation stage, 6. Heat exchanger evaporating alcohol at first evaporative stage, 7. Buffer tank for first evaporative stage, 8. Heat exchanger evaporating alcohol at second evaporative stage, 9. Circulation tank for second evaporative stage, 10. Buffer tank for second evaporative stage, 11. Condensed alcohol lock for first evaporative stage, 12. Condensed alcohol lock for second evaporative stage, 13. Evaporative system protection lock, 14-18 Glycerin fraction hour separators, 19. Ester phase buffer hour tank, 20. Disc centrifuge (vertical) - centrifugal separation of the glycerin phase and ester phase, 21-23. Multi-functional separation tanks; 24-hours separators of glycerin fraction, extractors for water extraction, 24. Disc centrifuge (vertical) - centrifugal separation of glycerin phase and ester phase, 25. Final product tank; Fig. 2 separator with nitrogen bubbler grill (21-23).

### Example 1. Preparation and purification of a mixture of DHA, EPA and ALA ethyl esters

The process of removing heavy metal compounds and organic impurities takes place in several stages:
a) spontaneously, in the process of creating phases arising in the process of alkaline transetrification (product phase and glycerol phase),
b) by gravitational separation of phases in hour separators,
c) by centrifugal separation in disc centrifuges,
d) through water extraction in 24-hours separators,
e) by gravity and centrifugal separation of the extractant (water) and the extracted phase (ester).

The diagram in Fig. 1 is an illustrative installation performing the above steps.

First, an alkaline transesterification with ethanol of higher fatty acids contained in the form of glycerides in edible oils should be carried out. For this purpose, a 13-16 molar excess of ethanol is preferably used. After complete chemical reaction (about 30 minutes) in the reactor system (3,4), ethanol should be evaporated under reduced pressure, preferably at absolute pressure from 0.02 bar to 0.05 bar in a closed loop system (7, 8, 9). The evaporation process ends when the ethanol concentration in the post-reaction mixture is below 0.1% by weight. The mixture prepared in this way is initially separated by gravity separation into the glycerine phase and the product phase for 150 minutes in hour separators (14-18). The glycerol phase (as the one with higher density) is discharged from the separator by the bottom connector. The product phase remaining in the separator is directed to the 24-hours separator (21-22) through a disc centrifuge (vertical) (20), where the mixture is separated again within 24 hours. The residues of the glycerin phase are again discharged by the bottom connector, and then the remaining ester phase is mixed with water in a ratio of 1:1 by volume and both phases are mixed with nitrogen gas. After an hour of extraction, the water phase is discharged from the bottom connection of the 24-hours separator (21-23) and directed again to the disk centrifuge to clean the product phase from the water phase (24). After this treatment, the product is ready for further use and is directed to the final product tank (25).

For the synthesis of esters, a composition containing a mixture of fatty acids with a high content of polyunsaturated omega fatty acids of fish and vegetable origin was used, with the following composition of omega fatty acids:
- Alpha-linolenic acid ALA (from 1% to 50% wt.)
- EPA eicosapentaenoic acid (from 1% to 40% wt.)
- DHA docosahexaenoic acid (from 1% to 40% wt.)
in various qualitative and quantitative combinations in the percentage ranges as above.

Because easily available vegetable oils are often characterized by a very high level of ALA (for example, linseed oil can contain up to nearly 70% ALA), it is easy to obtain the desired level of this acid in the mixture used for ester synthesis.

When using natural fish oils as raw materials, it is recommended to use the commercially available concentrated forms of EPA (eicosapentaenoic acid) and DHA (docosahexaenoic acid) to obtain higher concentrations of EPA and DHA in the oil mixture than in used fish oils. These can be, for example, preparations from Polaris - Functional lipids and KinOmega.

The fatty acid content of these oils is up to 80% EPA and up to 80% DHA.

For example, Polaris - Functional lipids offers oils with the following composition:
- Omegavie^{®} 4020 TG / EE Qualitysilver^{®} * - EPA 40%/DHA 20%
- Omegavie^{®}DHA 80 TG Qualitysilver^{®} - DHA 70%

The KinOmega company offers oils with the following composition:
KinOmega E80 TG - EPA80%
KinOmega 7010 TG - EPA70% + DHA10%
KinOmega D80 TG - DHA80%
KinOmega D75 TG - EPA5% + DHA75%.

In order to confirm the effectiveness of the method according to the invention, the level of selected impurities was analyzed in various batches of raw materials consisting of unsaturated animal (fish) and vegetable fats.

Laboratory determinations were carried out in accredited research laboratories using the methods recommended in the standards specifying the methods of measuring contaminants in food products. For individual impurities, their level was determined using the following methods:
Heavy metals (the recommended method is inductively coupled plasma mass spectrometry (ICP-MS):
- Cadmium: measurement by the method according to PN-EN 15763:2010,
- Arsenic: measurement by the method according to PN-EN 15763:2010,
- Lead: measurement by the method according to PN-EN 15763: 2010,
- Mercury: measurement by the method according to PB-30/CVAAS standard ed. V dated 09/18/2012

Dioxins: Dioxins/Furans/dioxin-like PCBs/indicator PCBs: measurement by the method according to NL/22a, NL/22b (EU 2017/664)
Pesticides: Pesticides screening: measurement by the method according to LMBG-00.00-34:1999 standard (DFG S19) excluding the E9 module.

As a result of the measurements, it was found that the composition of the raw material used contained the sum of polychlorinated dioxins and furans in an amount not exceeding 2.052 pg/g fat, the sum of polychlorinated biphenyls in an amount not exceeding 3.734 pg/g fat, the sum of polychlorinated dioxins, furans and biphenyls in an amount not exceeding 5.786 pg/g fat, the sum of polychlorinated biphenyls (ICES-6) in an amount not exceeding 29.47 ng/g fat, heavy metals selected from the group consisting of: arsenic, cadmium, lead, mercury, with arsenic in an amount not exceeding 1.35 mg/kg, cadmium in an amount not exceeding 0.002 mg/kg, lead in an amount not exceeding 0.05 mg/kg, mercury in an amount not exceeding 0.0023 mg/kg.

At the same time, analogous measurements were made of the composition and level of selected impurities in ethyl esters prepared from the analyzed raw materials by the method of the invention.

The composition according to the invention contained a mixture of fatty acid ethyl esters with the following composition of omega fatty acids:
- ALA alpha-linolenic acid ethyl ester (from 1% to 50% wt.)
- EPA eicosapentaenoic acid ethyl ester (from 1% to 40% wt.)
- DHA docosahexaenoic acid ethyl ester (from 1% to 40% wt.)
in various qualitative and quantitative combinations, determined by the composition of the raw material, in the percentage ranges specified above. At the same time, it was found that there was no change in the configuration of multiple bonds from cis to trans during the production of ethyl esters.

Regarding the level of selected impurities, it was found that the ester composition obtained by the method of the invention contains: the sum of polychlorinated dioxins and furans in an amount not exceeding 0.158 pg/g fat, the sum of polychlorinated biphenyls in an amount not exceeding 0.159 pg/g fat, the sum of polychlorinated derivatives of dioxin, furans and biphenyls in an amount not exceeding 0.317 pg/g fat, the sum of polychlorinated biphenyls (ICES-6) in an amount not exceeding 14.67 ng/g fat, heavy metals selected from the group consisting of: arsenic, cadmium, lead, mercury, with arsenic in an amount not exceeding 0.010 mg/kg, cadmium in an amount not exceeding 0.0010 mg/kg, lead in an amount not exceeding 0.010 mg/kg, mercury in an amount not exceeding 0.0006 mg/kg.

The results of measurements of selected impurities in the sample batches of raw materials used for transesterification and constituting, in both cases, the mixtures of fish and vegetable oils with the content of ALA, EPA and DHA as defined above, as well as in the ethyl esters obtained from them, are presented in Table 2A - 2B.

**Table 2A. The content of trace impurities (heavy metals, dioxins) in the purified product according to the invention, compared to the content of impurities in the raw materials. Oil I and oil II are raw materials from two different suppliers.**

| **Designation** | | **Oil I** | **Oil II** | **Ethyl esters** | **Unit** |
|---|---|---|---|---|---|
| Dioxins | WHO-PCDD/F-TEQ | 2.052 | 0.512 | 0.158 ± 0.024 | pg/g fat |
| | WHO-PCB-TEQ | 3.734 | 0.967 | 0.159 ± 0.032 | pg/g fat |
| | WHO-PCDD/F-PCB-TEQ | 5.786 | 1.261 | 0.317 ± 0.079 | pg/g fat |
| | PCB total (ICES - 6) | 24.57 | 29.47 | 14.67 ± 2.93 | ng/g fat |
| Heavy metals | Arsenic | 1.35 | < 0.09 | < 0.01 | mg/kg |
| | Cadmium | < 0.0010 | < 0.002 | < 0.001 | mg/kg |
| | Lead | < 0.010 | < 0.05 | < 0.01 | mg/kg |
| | Mercury | 0.0019 | 0.0023 ± 0.0002 | 0.0006 ± 0.0001 | mg/kg |

| | | | | | |
|---|---|---|---|---|---|
| **Legend** **WHO-PCDD/F-TEQ**/g sample (fat) - sum of dioxins **WHO-PCDD/F-PCB-TEQ**/g sample (fat) - sum of dioxins and polychlorinated biphenyls with dioxin-like properties **WHO-PCB-TEQ** - sum of polychlorinated biphenyls **PCDD** - congeners of polychlorinated dibenzo-p-dioxins **PCDF-** polychlorinated dibenzo-furans | | | | | |

**Table 2B. The content of trace impurities (heavy metals, dioxins) in the purified product according to the invention, compared to the content of impurities in the raw materials. Oil I and Oil II mean exemplary fish oil and vegetable oil used as raw materials. Two oil mixtures were used to produce two mixtures of esters.**

| | **Designation** | | **Oil I Fish oil** | **Oil II Vegetable oil** | **Mixture I** | **Mixture II** | **Mixture I Esters** | **Mixture II Esters** | **Unit** |
|---|---|---|---|---|---|---|---|---|---|
| Fatty acid content | | ALA | 1,93 | 55 | 50 | 13 | 50 | 13 | |
| | | EPA | 10,22 | 0 | 1 | 8 | 1 | 8 | % |
| | | DHA | 13,65 | 0 | 1 | 11 | 1 | 11 | |
| Dioxins | | WHO-PCDD/F-TEQ | 0,512 | 0,589 | 0,581 | 0,527 | 0,158 ± 0,024 | 0,158 ± 0,024 | pg/g fat |
| | | WHO-PCB-TEQ | 0.967 | 0,797 | 0,814 | 0,933 | 0,114 ± 0,023 | 0,159 ± 0,032 | pg/g fat |
| | | WHO-PCDD/F-PCB-TEQ | 1,261 | 1,244 | 1,246 | 1,258 | 0,272 ± 0,068 | 0,317 ± 0,079 | pg/g fat |
| | | Sum of PCB (ICES - 6) | 29,47 | 1,386 | 4,195 | 23,853 | <0,6 | 14,67 ± 2,93 | ng/g fat |
| Heavy metals | | Arsenic | <0,09 | 0,68 | 0,62 | 0,208 | <0,01 | <0,01 | mg/kg |
| | | Cadmium | <0,002 | <0,005 | <0,005 | <0,003 | <0,001 | <0,001 | mg/kg |
| | | Lead | <0,05 | <0,05 | <0,05 | <0,05 | <0,01 | <0,01 | mg/kg |
| | | Mercury | 0,0023 ± 0,0002 | 0,007 | <0,007 | 0,003 | <0,0006 | 0,0006 ±0,0001 | mg/kg |

| | | | | | | | | | |
|---|---|---|---|---|---|---|---|---|---|
| Legend: Mixture I - Oil I (10%) + Oil II (90%) Mixture II - Oil I (80%) + Oil II (20%) | | | | | | | | | |

## Claims

1. A method of obtaining a purified composition containing unsaturated fatty acid esters obtained from a transesterification reaction of a natural oil containing unsaturated fatty acid triglycerides with ethyl alcohol, **characterized in that** the purification of the reaction mixture is carried out in successive stages:
a) after the transesterification reaction, the alcohol is evaporated from the reaction mixture, wherein the evaporation process is carried out at a temperature not exceeding 49 °C for not more than an hour,
b) the ester phase obtained in step (a) is separated,
c) the ester phase is purified from the catalyst residues, soaps and glycerin in the centrifugation process,
d) the ester phase is mixed with water in a volume ratio of 1:1, with nitrogen gas flow through the resulting two-phase system at 5-10 m³/h for one hour, then the nitrogen flow is stopped and the mixture is left to separate into two phases, then the aqueous phase is separated.
e) the purified ester mixture is clarified by centrifugation.

2. The method according to claim 1, **characterized in that** the evaporation is carried out in a two-stage evaporative module.

3. The method according to claim 1, **characterized in that** the ester phase is mixed with water in the 24-hours separator.

4. The method according to claim 1, **characterized in that** a mixture of fish and vegetable oil is used to produce esters.

5. Purified composition containing ethyl esters of unsaturated fatty acids obtained by transesterification of a natural oil containing unsaturated fatty acid triglycerides with ethyl alcohol, **characterized in that** it contains a mixture of ethyl esters of fatty acids with the following composition of cis-omega fatty acids:
- ALA alpha-linolenic acid ethyl ester in an amount of 1% to 50% wt.,
- EPA eicosapentaenoic acid ethyl ester in an amount of 1% to 40% wt.,
- DHA docosahexaenoic acid ethyl ester in an amount of 1% to 40% wt.,
wherein it is essentially free of dioxins, biphenyls, their derivatives, and heavy metals contaminants, and contains a sum of dioxins in an amount not exceeding 0.158 pg/g fat, a sum of polychlorinated biphenyls in an amount not exceeding 0.159 pg/g fat, a sum of polychlorinated dioxins and biphenyls in an amount not exceeding 0.317 pg/g fat, a sum of polychlorinated biphenyls (ICES-6) in an amount not exceeding 14.67 ng/g fat, and heavy metals selected from the group consisting of: arsenic, cadmium, lead, mercury, with arsenic in an amount not exceeding 0.01 mg/kg, cadmium in an amount not exceeding 0.001 mg/kg, lead in an amount not exceeding 0.01 mg/kg, mercury in an amount not exceeding 0.0006 mg/kg.

6. Composition according to claim 5, **characterized in that** it comprises ethyl esters of unsaturated fatty acids derived from a mixture of fish and vegetable oil.

## Patentansprüche

1. Verfahren zur Gewinnung einer gereinigten Zusammensetzung, die ungesättigte Fettsäureester enthält, welche aus einer Umesterungsreaktion eines natürlichen Öls, das ungesättigte Fettsäuretriglyceride enthält, mit Ethylalkohol erhalten wurden, **dadurch gekennzeichnet, dass** die Reinigung des Reaktionsgemischs in aufeinanderfolgenden Stufen durchgeführt wird:
a) nach der Umesterungsreaktion wird der Alkohol aus dem Reaktionsgemisch verdampft, wobei der Verdampfungsvorgang bei einer Temperatur, die 49°C nicht übersteigt, während nicht mehr als einer Stunde durchgeführt wird,
b) die in Schritt (a) erhaltene Esterphase wird abgetrennt,
c) die Esterphase wird im Zentrifugationsverfahren von den Katalysatorresten, Seifen und Glycerin gereinigt,
d) die Esterphase wird in einem Volumenverhältnis von 1:1 mit Wasser gemischt, wobei eine Stunde lang Stickstoffgas mit 5-10 m³/h durch das resultierende Zweiphasensystem strömt, dann wird der Stickstoffstrom unterbrochen, und das Gemisch wird sich in zwei Phasen auftrennen gelassen, dann wird die wässrige Phase abgetrennt,
e) das gereinigte Estergemisch wird durch Zentrifugation geklärt.

2. Verfahren gemäß Anspruch 1, **dadurch gekennzeichnet, dass** die Verdampfung in einem zweistufigen Verdampfungsmodul durchgeführt wird.

3. Verfahren gemäß Anspruch 1, **dadurch gekennzeichnet, dass** die Esterphase in dem 24-Stunden-Separator mit Wasser gemischt wird.

4. Verfahren gemäß Anspruch 1, **dadurch gekennzeichnet, dass** ein Gemisch von Fisch- und Pflanzenöl verwendet wird, um Ester zu produzieren.

5. Gereinigte Zusammensetzung, die Ethylester von ungesättigten Fettsäuren enthält, welche durch Umesterung eines natürlichen Öls, das ungesättigte Fettsäuretriglyceride enthält, mit Ethylalkohol erhalten wurden, **dadurch gekennzeichnet, dass** sie ein Gemisch von Ethylestern von Fettsäuren mit der folgenden Zusammensetzung an cis-omega-Fettsäuren enthält:
- ALA alpha-Linolensäureethylester in einer Menge von 1 bis 50 Gew.-%,
- EPA Eicosapentaensäureethylester in einer Menge von 1 bis 40 Gew.-%,
- DHA Docosahexaensäureethylester in einer Menge von 1 bis 40 Gew.-%,
wobei sie im Wesentlichen frei von Dioxinen, Biphenylen, ihren Derivaten und Schwermetallkontaminanten ist und Folgendes enthält: eine Summe von Dioxinen in einer Menge, die 0,158 pg/g Fett nicht übersteigt, eine Summe von polychlorierten Biphenylen in einer Menge, die 0,159 pg/g Fett nicht übersteigt, eine Summe von polychlorierten Dioxinen und Biphenylen in einer Menge, die 0,317 pg/g Fett nicht übersteigt, eine Summe von polychloriertem Biphenyl (ICES-6) in einer Menge, die 14,67 ng/g Fett nicht übersteigt, und Schwermetalle, die aus der Gruppe ausgewählt sind, die aus Arsen, Cadmium, Blei und Quecksilber besteht, mit Arsen in einer Menge, die 0,01 mg/kg nicht übersteigt, mit Cadmium in einer Menge, die 0,001 mg/kg nicht übersteigt, mit Blei in einer Menge, die 0,01 mg/kg nicht übersteigt, mit Quecksilber in einer Menge, die 0,0006 mg/kg nicht übersteigt.

6. Zusammensetzung nach Anspruch 5, **dadurch gekennzeichnet, dass** sie Ethylester von ungesättigten Fettsäuren umfasst, die aus einem Gemisch von Fisch- und Pflanzenöl stammen.

## Revendications

1. Procédé d'obtention d'une composition purifiée contenant des esters d'acides gras insaturés obtenus à partir d'une réaction de transestérification d'une huile naturelle contenant des triglycérides d'acides gras insaturés avec de l'alcool éthylique, **caractérisé en ce que** la purification du mélange de réaction est effectuée en étapes successives :
a) après la réaction de transestérification, l'alcool est évaporé du mélange de réaction, dans lequel le processus d'évaporation est effectué à une température ne dépassant pas 49 °C pendant pas plus d'une heure,
b) la phase ester obtenue à l'étape a) est séparée,
c) la phase ester est purifiée concernant les résidus de catalyseur, les savons et la glycérine dans le processus de centrifugation,
d) la phase ester est mélangée à de l'eau dans un rapport volumétrique de 1:1, avec un flux d'azote gazeux traversant le système biphasé résultant à 5 à 10 m³/h pendant une heure, puis le flux d'azote est arrêté et on laisse le mélange se séparer en deux phases, puis la phase aqueuse est séparée,
e) le mélange d'esters purifié est clarifié par centrifugation.

2. Procédé selon la revendication 1, **caractérisé en ce que** l'évaporation est effectuée dans un module d'évaporation à deux étages.

3. Procédé selon la revendication 1, **caractérisé en ce que** la phase ester est mélangée avec de l'eau dans le séparateur 24 heures.

4. Procédé selon la revendication 1, **caractérisé en ce qu'**un mélange d'huile de poisson et d'huile végétale est utilisé pour produire des esters.

5. Composition purifiée contenant des esters éthyliques d'acides gras insaturés obtenus par transestérification d'une huile naturelle contenant des triglycérides d'acides gras insaturés avec de l'alcool éthylique, **caractérisée en ce qu'**elle contient un mélange d'esters éthyliques d'acides gras avec la composition suivante d'acides gras cis-oméga :
- ester éthylique d'acide alpha-linolénique ALA dans une quantité de 1 % à 50 % en poids,
- ester éthylique d'acide éicosapentaénoïque EPA dans une quantité de 1 % à 40 % en poids,
- ester éthylique d'acide docosahexaénoïque DHA dans une quantité de 1 % à 40 % en poids,
dans laquelle elle est essentiellement exempte de dioxines, de biphényles, de leurs dérivés et de contaminants de métaux lourds, et contient une somme de dioxines dans une quantité ne dépassant pas 0,158 pg/g de graisse, une somme de biphényles polychlorés dans une quantité ne dépassant pas 0,159 pg/g de graisse, une somme de dioxines et de biphényles polychlorés ne dépassant pas 0,317 pg/g de graisse, une somme de biphényles polychlorés (ICES-6) dans une quantité ne dépassant pas 14,67 ng/g de graisse, et des métaux lourds choisis dans le groupe consistant en : l'arsenic, le cadmium, le plomb, le mercure, avec l'arsenic dans une quantité ne dépassant pas 0,01 mg/kg, le cadmium dans une quantité ne dépassant pas 0,001 mg/kg, le plomb dans une quantité ne dépassant pas 0,01 mg/kg, le mercure dans une quantité ne dépassant pas 0,0006 mg/kg.

6. Composition selon la revendication 5, **caractérisée en ce qu'**elle comprend des esters éthyliques d'acides gras insaturés dérivés d'un mélange d'huile de poisson et d'huile végétale.
